(19) Europäisches Patentamt
European Patent Office
Office européen des brevets

(11) **EP 3 659 579 A1**

(12) **EUROPEAN PATENT APPLICATION**
published in accordance with Art. 153(4) EPC

(43) Date of publication:
**03.06.2020 Bulletin 2020/23**

(51) Int Cl.:
**A61K 8/44** *(2006.01)*    **A61K 8/06** *(2006.01)*
**A61K 8/19** *(2006.01)*    **A61K 8/25** *(2006.01)*
**A61K 8/37** *(2006.01)*    **A61K 8/891** *(2006.01)*
**A61K 8/894** *(2006.01)*    **A61Q 19/00** *(2006.01)*

(21) Application number: **18838603.1**

(22) Date of filing: **24.07.2018**

(86) International application number:
**PCT/JP2018/027606**

(87) International publication number:
**WO 2019/022041 (31.01.2019 Gazette 2019/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.07.2017   JP 2017142666**

(71) Applicant: **Kokyu Alcohol Kogyo Co., Ltd.**
**Narita-shi**
**Chiba 287-0225 (JP)**

(72) Inventors:
• **OMURA, Takayuki**
  **Narita-shi**
  **Chiba 287-0225 (JP)**
• **KAWAI, Kiyotaka**
  **Narita-shi**
  **Chiba 287-0225 (JP)**

(74) Representative: **Cabinet Nuss**
**10, rue Jacques Kablé**
**67080 Strasbourg Cedex (FR)**

(54) **W/O EMULSION**

(57)    Provided is an W/O emulsion that is stable and has an excellent feeling on use.
    An W/O emulsion including component (A) : dibutyl lauroyl glutamide, component (B) : dibutyl ethylhexanoyl glutamide, component (C) : an organically modified clay mineral, component (D) : a polyether-modified silicone, component (E) : silicone oil, and component (F): ester oil.

EP 3 659 579 A1

## Description

[Technical Field]

The present invention relates to a W/O type emulsion that is used for cosmetics etc.

[Background Art]

A w/o type emulsion comprising silicone oil is known as an emulsion with excellent slipperiness, and it is proposed to use a polyether-modified silicone and an organically modified clay mineral to suppress oiliness and stickiness (Patent Document 1).
Such W/O type emulsion is excellent in slipperiness, but inferior in moist feeling and emollient property.
An ester oil is known as an oil agent for imparting emollient property, but there was a problem that when an ester oil was added to a W/O emulsion mainly composed of silicone oil, a stable emulsion could not be obtained. It has been proposed to use a combination of a cross-linked polyether-modified silicone and a cross-linked polyether-modified silicone containing alkyl groups as a composition that enables the addition of an ester oil (Patent Document 2), however, this formulation does not use an organically modified clay mineral; there is still a problem in obtaining a stable W/O type emulsion which is a system employing a polyether-modified silicone and an organically modified clay mineral and in which a large amount of an ester oil is blended.
Meanwhile, it has been proposed to stabilize a water-in-silicone type emulsion by means of a glutamide compound (Patent Document 3). However, this is not a water-in-oil type emulsion having a high emollient feeling, and a water-in-oil type emulsion comprising an ester oil has not been studied at all.

[Prior Art Literatures]

[Patent Literatures]

[Patent literature 1] JP H08-268831 A
[Patent literature 2] JP 2002-275028 A
[Patent literature 3] WO 2012/091823

[Summary of the Invention]

[Problems to Be Solved by the Invention]

In view of the above-described problems, the present inventors have studied on the object of providing a W/O type emulsion in which an ester oil can be blended and which has excellent stability.

[Means for Solving the Problems]

During diligent researches, the present inventors have found that it is possible to blend an ester oil in a W/O type emulsion mainly composed of a silicone oil by combining dibutyl lauroyl glutamide and dibutyl ethylhexanoyl glutamide with a polyether-modified silicone and an organically modified clay mineral, and as a result of further studies, they have completed the present invention.

[1] A W/O type emulsion comprising

Component (A): dibutyl lauroyl glutamide,
Component (B): dibutyl ethylhexanoyl glutamide,
Component (C): an organically modified clay mineral,
Component (D): a polyether-modified silicone,
Component (E): a silicone oil, and
Component (F): an ester oil.

[2] The emulsion according to [1] comprising 5% by mass or more of the ester oil.
[3] The emulsion according to [1] or [2], wherein the mass ratio of the ester oil / the silicone oil is 0.5 or more.

[4] The emulsion according to [3], wherein the mass ratio of the ester oil / the silicone oil is 1.0 or more.

[5] The emulsion according to any one of [1] to [4] comprising 3.5% by mass or more of the silicone oil.

[6] The emulsion according to any one of [1] to [5] wherein the component (D) comprises a non-crosslinked polyether-modified silicone.

[7] The emulsion according to any one of [1] to [6] comprising

0.1 to 20.0% by mass of the component (A),
0.1 to 10.0% by mass of the component (B),
0.5 to 5.0% by mass of the component (C), and
0.5 to 5.0% by mass of the component (D).

[8] The emulsion according to any one of [1] to [7] which is in the form of balm.

[9] The emulsion according to any one of [1] to [8] which is a cosmetic.

**[Advantageous Effects of the Invention]**

By combining dibutyl lauroyl glutamide, dibutyl ethylhexanoyl glutamide, a polyether-modified silicone and an organically modified clay mineral, it has become possible to provide a W/O type emulsion containing a silicone oil with suppressed separation as well as with storage stability, even when the ester oil is blended at high level.

Further, since it becomes possible to blend the ester oil at high level, it is possible to provide a W/O type emulsion with excellent feeling of use, which can provide moist feeling and emollient property in addition to the properties of spreadability on the skin and light feeling without stickiness after the application.

Furthermore, it is possible to prepare an emulsion in the form of cream to solid (balm), and thus it is possible to provide cosmetics in a shape suitable for the intended use.

**[Embodiments for Carrying Out the Invention]**

<W/O type emulsion>

A "W/O type emulsion" is a water-in-oil type emulsion, that is, an emulsion in which an aqueous component is dispersed in a continuous phase containing an oil component.

In one embodiment of the present invention, the W/O type emulsion can be used for any purpose, but typically can be used for external preparations such as pharmaceuticals, quasi-drugs, and cosmetics. Since the emulsion of the present invention has a characteristic texture and a characteristic feeling upon the application, it is preferably used as an external preparation for the skin. In one embodiment of the invention, the W/O type emulsion is preferably a cosmetic, particularly preferably a cosmetic for the skin.

The W/O type emulsion of the present invention can be used in various forms of products, e.g. a pharmaceutical such as an external preparation comprising a medicament for the skin; a quasi-drug such as a cosmeceutical; a skin care cosmetic such as a moisturizing cream, a whitening cream, an anti-aging cream, a moisturizing serum, a whitening serum, an anti-aging serum, a moisturizing balm, a whitening balm, an anti-aging balm, a sunscreen cream, a sunscreen balm, a moisturizing milky lotion in the form of cream, a whitening milky lotion in the form of cream, an anti-aging serum in the form of cream; a makeup cosmetic such as a foundation in the form of cream, a foundation in the form of balm, a makeup base in the form of cream, a makeup base in the form of balm, a concealer in the form of cream, and a concealer in the form of balm.

<Component A: Dibutyl lauroyl glutamide (GP-1)>

Dibutyl lauroyl glutamide is an amino acid based oil gelling agent and commercially available under the product name "GP-1 (Name of the component: N-lauroyl-L-glutamic acid dibutylamide)" (Ajinomoto Co. Inc.). In this specification, dibutyl lauroyl glutamide is also referred to as "GP-1".

The blending amount of dibutyl lauroyl glutamide in the W/O type emulsion can be appropriately adjusted depending on the type and amount of the oil component as well as the viscosity required for the emulsion, and is not particularly limited. However, from the viewpoint of ensuring usability and stability, the amount may be 0.1 to 20.0% by mass, preferably 0.5 to 15.0% by mass, more preferably 1.0 to 10.0% by mass.

<Component B: Dibutyl ethylhexanoyl glutamide(EB-21)>

Dibutyl ethylhexanoyl glutamide is an amino acid based oil gelling agent and commercially available under the product name "EB-21 (Name of the component: N-2-ethyl hexanoyl-L-glutamic acid dibutylamide)" (Ajinomoto Co., Inc.) .

The blending amount of dibutyl ethylhexanoyl glutamide in the W/O type emulsion can be appropriately adjusted depending on the type and amount of the oil component as well as the viscosity required for the emulsion, and is not particularly limited. However, from the viewpoint of ensuring usability and stability, the amount may be 0.1 to 10.0% by mass, preferably 0.2 to 8.0% by mass, more preferably 0.3 to 5.0% by mass.

Dibutyl lauroyl glutamide and dibutyl ethylhexanoyl glutamide are also commercially available as a premix product, and such premix can be used in the present invention. For example, it is commercially available under the product names AJK-OD2046 (Octyldodecanol, Dibutyl lauroyl glutamide, Dibutyl ethylhexanoyl glutamide), and AJK-IS(Isostearic acid, Dibutyl lauroyl glutamide, Dibutyl ethylhexanoyl glutamide) from Kokyu Alcohol Kogyo Co., Ltd.

<Component C: Organically modified clay mineral>

An organically modified clay mineral is the one in which exchangeable cation(s) interposed between crystal layers of a water-swellable clay mineral (e.g. montmorillonite, saponite, hectorite, bentonite, etc.) with an organic polar compound or an organic cation (for example, a quaternary ammonium cationic surfactant) .

The organically modified clay mineral used in the present invention is not particularly limited as long as it is a component generally used in cosmetics and the like. Commercially available organically modified clay minerals can be used, and they are commercially available from Elementis Specialities Inc. under the product names, for example, BENTONE 27V (Stearalkonium Hectorite), BENTONE 27VCG (Stearalkonium Hectorite), BENTONE 38V (Disteardimonium Hectorite), BENTONE 38VCG (Disteardimonium Hectorite).

Further, the organically modified clay mineral is also commercially available as a premix in which it is dissolved in a silicone oil, an ester oil and/or other oil agent, and such premix can also be used in the present invention, and it is commercially available under the following product names from Elementis Specialities Inc.:
BENTONE GEL 1002V (Cyclopentasiloxane, Disteardimonium Hectorite, Propylene Carbonate), BENTONE GEL ABO V (Crambe Abyssinica Seed Oil, Stearalkonium Hectorite, Propylene Carbonate), BENTONE GEL CAO V (Castor Oil, Stearalkonium Hectorite, Propylene Carbonate), BENTONE GEL EUG V (Octyldodecanol, Disteardimonium Hectorite, Propylene Carbonate), BENTONE GTCC V (Caprylic/Capric Triglyceride, Stearalkonium Hectorite, Propylene Carbonate), BENTONE HSO V (Caprylic/Capric Triglyceride, Stearalkonium Hectorite, Propylene Carbonate), BENTONE IHD V (Isohexadecane, Disteardimonium Hectorite, Propylene Carbonate), BENTONE IPM V (Isopropyl Myristate, Stearalkonium Hectorite, Propylene Carbonate), BENTONE ISD V (Isododecane, Disteardimonium Hectorite, Hectorite), BENTONE LOI V (Lanolin Oil, Isopropyl Palmitate, Stearalkonium Hectorite, Propylene Carbonate), BENTONE MSO(V) (Meadowfoam Oil, Disteardimonium Hectorite, Propylene Carbonate), BENTONE NGD V (Neopentyl Glycol Diheptanoate, Disteardimonium Hectorite, Propylene Carbonate), BENTONE OLV V (Olive Fruit Oil, Stearalkonium Hectorite, Propylene Carbonate), BENTONE OMS V (C11-12 Isoparaffin, Disteardimonium Hectorite, Alcohol Denat), BENTONE PTIS V (Pentaerythrityl Tetraisostearate, Disteardimonium Hectorite, Propylene Carbonate), BENTONE SS71V (Petroleum Distillates, Disteardimonium Hectorite, Propylene Carbonate), BENTONE TMF V (Methyl Trimethicone, Disteardimonium Hectorite, Triethyl Citrate), BENTONE TN V (C12-15 Alkyl Benzoate, Stearalkonium Hectorite, Propylene Carbonate), BENTONE VS-5V(V) (Cyclopentasiloxane, Disteardimonium Hectorite, Alcohol Denat), BENTONE VS-5PC V(HV) (Cyclopentasiloxane, Disteardimonium Hectorite, Propylene Carbonate).

The blending amount of the organically modified clay mineral in the W/O type emulsion can be appropriately adjusted depending on the type and amount of the oil component as well as the viscosity required for the emulsion, and is not particularly limited, however, it may be 0.5 to 5.0% by mass, preferably 0.7 to 4.0% by mass, more preferably 1.0 to 3.0% by mass.

<Component D: Polyether-modified silicone>

A polyether-modified silicone is a polymer having a silicone chain as a main chain and a polyether chain introduced by modification.

The polyether-modified silicone used in the present invention is not particularly limited as long as it is a component generally used in cosmetics and the like.

The polyether-modified silicone may be liner or branched. Further, the polyether-modified silicone may be a crosslinked type in which a silicone chain as a main chain is crosslinked with a polyether chain. Further, the polyether-modified silicone may be a polyether-alkyl co-modified type having an alkyl chain.

The polyether-modified silicone can be produced by an ordinary method, but a commercially available product can also be used, or a premix product in which it is diluted with a silicone oil or the like can also be used.

The type of the polyether-modified silicone used in the present invention can be appropriately selected depending on the properties required, for example, hardness and the like, and it can be used alone or two or more types can be used in combination. In one embodiment of the present invention, it is preferable to use a non-crosslinked polyether-modified silicone from the viewpoint of giving a rich feeling (thick feeling).

Examples of the non-crosslinked polyether-modified silicone include liner type polyether-modified silicones such as KF-6017 (PEG-10 Dimethicone), KF-6017P (PEG-10 Dimethicone), KF-6028 etc.: Branched type, Alkyl co-modified type polyether-modified silicones such as KF-6038 (Lauryl PEG-9 Polydimethylsiloxyethyl Dimethicone): Liner type, alkyl co-modified type polyether-modified silicones such as KF-6048 (Cetyl PEG/PPG-10/1 Dimethicone) etc. (From Shin-Etsu Chemical Co., Ltd.).

Examples of the crosslinked type polyether-modified silicone include standard type polyether-modified silicone cross-linked product such as KSG-210 (Dimethicone / (PEG-10/15)) Crosspolymer, Dimethicone), KSG-240 (Dimethicone / (PEG-10/15)) Crosspolymer, Cyclopentasiloxane) etc.: polyether-modified silicone cross-linked product having alkyl chain in its structure such as KSG-310 (PEG-15 / Lauryl Dimethicone) Crosspolymer, Mineral oil), KSG-320 ((PEG-15 / Lauryl Dimethicone) Crosspolymer, Isododecane), KSG-330 ((PEG-15 / Lauryl Dimethicone) Crosspolymer, Triethylhexanoin), KSG-340 ((PEG-10 / Lauryl Dimethicone) Crosspolymer, (PEG-15 / Lauryl Dimethicone) Crosspolymer), KSG-810 ((Lauryl Dimethicone/Polyglycerin-3) Crosspolymer, Mineral oil) etc.

Further, it is commercially available from Dow Corning Toray Co.,Ltd. under the product name of ES-5612 Formulation Aid (PEG-10 Dimethicone), BY-11-030 (PEG/PPG-19/19 Dimethicone, Cyclopentasiloxane), BY25-337 (PEG/PPG-19/19 Dimethicone, Hydrogenated Polyisobutene), BY22-008M (PEG/PPG-19/19 Dimethicone, Cyclopentasiloxane), 5200 Formulation Aid(Lauryl PEG/PPG-18/18 Methicone), ES-5300 Formulation Aid(LaurylPEG-10 Tris (Trimethylsiloxy) silylethyl Dimethicone), ES-5600 Silicone Glycerol Emulsifier (Cetyl Diglyceryl Tris (Trimethylsiloxy) silylethyl Dimethicone), EL-7040 Hydro Elastomer Blend (Caprylyl Methicone, (PEG-12 Dimethicone / PPG-20 Crosspolymer).

It is also possible to use Silsoft 900(PPG-12 Dimethicone), SF1528 (Cyclopentasiloxane, PEG/PPG-20/15 Dimethicone), SF1528 (Cyclopentasiloxane, PEG/PPG-20/15 Dimethicone) etc. from Momentive Performance Materials Inc.

The type of the polyether-modified silicone can be appropriately selected depending on the type and amount of the oil agent used, and it can be used alone or two or more types can be used in combination.

The blending amount of the polyether-modified silicone in the W/O type emulsion can be appropriately adjusted depending on the type and amount of the oil component as well as the viscosity required for the emulsion, and is not particularly limited, however, it may be 0.5 to 5.0% by mass, preferably 1.0 to 4.5% by mass, more preferably 2.5 to 4.0% by mass.

<Component E: Silicone oil>

A silicone oil used in the present invention is not particularly limited as long as it is a component generally used in cosmetics and the like, but it includes, for example, dimethyl silicone oil, volatile silicone, methylphenyl silicone oil, amino-modified silicone, alkyl-modified silicone, high polymerization dimethiconol, high polymerization amino gum, silicone gel, acrylic silicone, trimethylsiloxysilicate, ethyltrisiloxane, phenyl-modified silicone oil, fluoro-modified silicone resin, phenyl-modified silicone resin, silicone wax and the like.

The type of the silicone oil used in the present invention can be appropriately selected depending on properties required, for example, hardness and the like, and it can be used alone or two or more types can be used in combination.

For example, to prepare an emulsion in the form of balm, a silicone oil with a high kinematic viscosity is selected.

The blending amount of a silicone oil in the W/O type emulsion can be appropriately adjusted depending on the type and amount of the oil component as well as the hardness required for the emulsion, and is not particularly limited, however, from the viewpoint of compatibility with the ester oil, it may be 0.5 to 20.0% by mass, preferably 1.0 to 15.0% by mass, more preferably 2.0 to 10.0% by mass.

In one embodiment of the present invention, from the view point of emulsifying property, it is desirable that the blending amount of the silicone oil in the W/O type emulsion is 3.5% by mass or more, preferable 5.0% by mass or more, more preferably 7.0% by mass or more.

<Component F: Ester oil>

An ester oil used in the present invention is not particularly limited as long as it is a component generally used in cosmetics and the like, but it includes, for example, as a monoester oil, isononanoic acid ester such as isononyl isononanoate, isotridecyl isononanoate; 2-ethylhexanoic acid ester such as cetyl ethylhexanoate, hexyldecyl ethylhexanoate; myristic acid ester such as isopropyl myristate, isocetyl myristate, octyldodecyl myristate; isostearic acid ester such as ethyl isostearate, isopropyl isostearate, hexyldecyl isostearate, isostearyl isostearate, cholesteryl isostearate, phytosteryl isostearate, isostearyl isobutyrate; lactic acid ester such as isostearyl lactate, octyldodecyl lactate; oleic acid ester such as oleyl oleate, phytosteryl oleate, octyldodecyl oleate; neopentanoic acid ester such as isodecyl neopentanoate, isostearyl neopentanoate etc.; palmitate ester such as isopropyl palmitate, ethylhexyl palmitate; and others such as octyldodecyl neodecanoate, octyldodecyl ricinoleate, oleyl erucate, octyldodecyl erucate, lauroyl sarcosine isopropyl, and the like.

As diester oil, it includes diisobutyl adipate, diisopropyl adipate, diethylhexyl succinate, neopentyl glycol diisononanoate, neopentyl glycol diethylhexanoate, neopentyl glycol dicaprate, diisostearyl malate, diisopropyl dilinoleate, ethylene glycol dioctanoate, octyldodecyl stearoyloxystearate, diisopropyl sebacate, cholesteryl/octyldodecyl lauroyl glutamate, phytosteryl/octyldodecyl lauroyl glutamate, and the like.

As triester oil, it includes triethylhexanoin, trimethylolpropane triethylhexanoate, caprylic/capric triglyceride, triisostearin, trimethylolpropane triisostearate and the like.

As tetraester oil, it includes pentaerythrityl tetraethylhexanoate, pentaerythrityl tetraisostearate, and the like.

As polyester, it includes polyglycerin fatty acid ester such as polyglyceryl-2 isostearate, polyglyceryl-2 diisostearate, polyglyceryl-2 triisostearate, polyglyceryl-2 tetraisostearate.

As highly viscous ester oil, it includes isostearic acid hydrogenated castor oil, dimer dilinoleic acid hydrogenated castor oil, polyglyceryl-2 isostearate / dimer dilinoleate copolymer, phytosteryl / isostearyl / cetyl / stearyl / behenyl dimer dilinoleate, bis-behenyl / isostearyl / phytosteryl dimer dilinoleyl dimer dilinoleate, phytosteryl isostearyl dimer dilinoleate, dimer dilinoleyl hydrogenated rosinate, dimer dilinoleyl diisostearate, dimer dilinoleyl dimer dilinoleate, cholesteryl / behenyl / octyldodecyl lauroyl glutamate, phytosteryl / behenyl / octyldodecyl lauroyl glutamate, phytosteryl / decyltetradecyl myristoyl methyl alaninate, and the like.

The type of the ester oil used in the present invention can be appropriately selected depending on properties required, for example, hardness and the like, and it can be used alone or two or more types can be used in combination.

The blending amount of the ester oil in the W/O type emulsion can be appropriately adjusted depending on the type and amount of the oil component as well as the hardness required for the emulsion, and is not particularly limited, however, from the view point of exertion of the oil gelling property by the components (A) and (B), it may be 3.0 to 20.0% by mass, preferably 5.0 to 20.0% by mass, more preferably 7.0 to 20.0% by mass.

In one embodiment of the present invention, the blending amount of the ester oil in the W/O type emulsion, from the view point of providing emollient feeling, it is desirable that it is 5% by mass or more, preferably 7% by mass or more, more preferably 10% by mass or more, further more preferably 13% by mass or more.

In one embodiment of the present invention, from the view point of making an emulsion with higher emollient feeling, the mass ratio of the ester oil / the silicone oil is preferably 0.5 or more, more preferably 1.0 or more.

<Additional oil component>

In one embodiment of the present invention, oil component other than the silicone oil and the ester oil can be used. The additional oil component is not particularly limited as long as it is a component generally used in cosmetics and the like, but it includes, for example, an animal and vegetable fat and oil, a hydrocarbon oil, a higher fatty acid, a higher alcohol, and the like; and it can be used alone or two or more types can be used in combination.

Examples of the animal and vegetable fat and oil, and hydrogenated animal and vegetable fat and oil include avocado oil, perilla oil, olive oil, cacao butter, kaya oil, apricot kernel oil, hardened oil, wheat germ oil, sesame oil, rice germ oil, rice bran oil, sugarcane wax, sasanqua oil, safflower oil, shea butter, tung oil, cinnamon oil, soybean oil, tea seed oil, Camellia japonica seed oil, evening primrose oil, corn oil, rapeseed oil, germ oil, palm oil, palm kernel oil, castor oil, hardened castor oil, sunflower oil, grape oil, jojoba oil, macadamia nut oil, beeswax, cottonseed oil, cotton wax, Japan wax, montan wax, coconut oil, hardened coconut oil, peanut oil, lanolin, liquid lanolin, reduced lanolin, lanolin alcohol, hard lanolin, lanolin acetate, lanolin fatty acid isopropyl ester, hexyl laurate, and the like.

Examples of the hydrocarbon oil include ozokerite, squalane, squalene, ceresin, paraffin, paraffin wax, liquid paraffin (Mineral oil), pristane, polyisobutylene, polyisobutene, hydrogenated polyisobutene, microcrystalline wax, polyethylene wax, Vaseline, and the like.

Examples of the higher fatty acid include lauric acid, myristic acid, palmitic acid, stearic acid, isostearic acid, oleic acid, behenic acid, undecylenic acid, oleic acid, linoleic acid, linolenic acid, arachidonic acid, eicosapentaenoic acid (EPA), docosahexaenoic acid (DHA), isostearic acid and the like.

Examples of the higher alcohol include myristyl alcohol, cetanol, stearyl alcohol, arachidyl alcohol, behenyl alcohol, and hydrogenated rapeseed oil alcohol, and the like.

In one embodiment of the present invention, the proportion of the silicone oil and the ester oil in the oil components is 50% by mass or more, preferably 60% by mass or more, more preferably 70% by mass or more, further more preferably 80% by mass or more.

<Aqueous component>

The aqueous component used in the W/O type emulsion of the present invention is not particularly limited as long as it is a component generally used in cosmetics and the like, but it includes, for example, water such as purified water and ion-exchanged water; lower alcohols such as BG (1,3-butylene glycol), PG (propylene glycol), dipropylene glycol, polyethylene glycol, glycerin, and ethanol, and it can be used alone or two or more types can be used in combination.

In one embodiment of the present invention, the blending amount of the aqueous component in the W/O type emulsion is not particularly limited, but in view of the feeling of use as an external preparation, it may be 20 to 80% by mass, preferably 23 to 78% by mass, more preferably 25 to 76% by mass.

<Other surfactant>

In one embodiment of the present invention, additional surfactant other than the component (D) can be used, and a surfactant (emulsifier) suitable for a W/O type emulsion can be appropriately selected, and examples include steareth-11, polyglyceryl-3 diisostearate, polyglyceryl-2 triisostearate, PEG-60 hydrogenated castor oil, PEG-30 glyceryl triisostearate, Lauryl PEG-9 polydimethylsiloxyethyl dimethicone, and the like.

<Other component>

The emulsion of the present invention may comprise any components used for external preparations such as cosmetics and the like.

Examples of these additional components include UV absorbers such as ethylhexyl methoxycinnamate, hexyl diethylaminohydroxybenzoyl benzoate; additinal thickeners and gelling agents such as acrylates / C10-30 alkyl acrylate crosspolymer, dextrin palmitate, xanthan gum; stabilizers such as sodium chloride and sodium citrate; quality-maintaining components such as antioxidants and preservatives; pharmaceutical components and active components such as whitening agents, anti-wrinkle agents, and antioxidants; and fragrance.

<Method for producing the W/O type emulsion>

The W/O type emulsion of the present invention can be prepared by a conventional method. For example, it can be prepared by emulsifying an aqueous component and an oil component in a heated state, and then stirring and cooling to room temperature.

Hereinafter, the present invention will be described in more detail with reference to examples. However, the present invention is not limited to these examples, and various modifications may be made without departing from the technical idea of the present invention. In this specification, unless otherwise specified, % means % by mass.

[Examples]

Water in oil type emulsified moisturizing creams for skin having the formulations shown in Tables 1 and 2 were prepared by a conventional method, and the stability and usability were evaluated by the following methods. The results are shown in Tables 1 and 2.

<Stability>

1. Hardness on the next day of the preparation, and hardness after storage at 25 °C for 1 month

The hardness was measured by placing the sample in a thermostat at 25 °C for 24 hours, and the measurement was made on the sample at room temperature of 25 °C using SHIMADZU EZ TEST EZ-SX with a needle-type jig having a diameter of 10 mm and a length of 30 mm at a speed of 10 mm / min and a stroke of 10 mm, and the maximum value of the hardness was defined as the hardness value of the sample.

2. Visual evaluation of appearance after 1 month storage at 50 °C for 1 month

The appearance of the sample was visually determined, and the stability was evaluated.
The case where no problems such as separation and discoloration were confirmed was evaluated as ○.

<Form>

On the next day of the preparation, whether it was in the form of cream or balm was determined.
It should be noted that the samples which were visually judged to be in the form of balm had hardness higher than 0.5 on the next day of the preparation.

<Usability>

An actual use test was conducted by a female professional panel (10 persons), and the test samples were evaluated for stickiness, spreadability on the skin, and moistness 3 hours after the application.

The evaluation method is as follows.

Usability (the feeling of use) was evaluated by a female professional panel (10 persons) according to the following evaluation criteria.

[Evaluation criteria for the feeling of use]

Evaluation was made for each of the following items: spreadability on the skin, stickiness after the application, and moistness 3 hours after the application.

With respect to the spreadability on the skin and the stickiness after the application, they were judged whether or not it was good by the sensory evaluation by the subject. With respect to the moistness 3 hours after the application, the skin condition of 10 persons of the female professional panel before and after the actual use test were evaluated by measuring the water content of the stratum corneum (arbitrary unit; au) with a Courage + Khazaka electronic from GmbH, Corneum moisture meter, Corneometer CM825, and a case where the value increased by 10.0 au or more before and after the actual use was evaluated as good.

In Table 1, the evaluation results are shown based on the following criteria.

◎: Good for 9 or more persons
○: Good for 7 to 8 persons
∆: Good for 3 to 6 persons
×: Good for 2 or less persons

[Table 1]

| | Name of the raw materials | Working examples | | | | | | | | | | | | |
|---|---|---|---|---|---|---|---|---|---|---|---|---|---|---|
| | | 1 | 2 | 3 | 4 | 5 | 6 | 7 | 8 | 9 | 10 | 11 | 12 | 13 |
| Aqueous component | Ion-exchanged water | Balance | Balance | Balance | Balance | Balance | Balance | Balance | Balance | Balance | Balance | Balance | Balance | Balance |
| | Glycerin | 13. 0 | 13. 0 | 13. 0 | 13. 0 | 13. 0 | 13. 0 | 13. 0 | 13. 0 | 13. 0 | 13. 0 | 13. 0 | 13. 0 | 13. 0 |
| | 1,3-butylene glycol | 2.0 | 2.0 | 2.0 | 2.0 | 2.0 | 2.0 | 2.0 | 2.0 | 2.0 | 2.0 | 2.0 | 2.0 | 2.0 |
| | Polyethylene Glycol #1540 | 1.0 | 1.0 | 1.0 | 1.0 | 1.0 | 1.0 | 1.0 | 1.0 | 1.0 | 1.0 | 1.0 | 1.0 | 1.0 |
| Component (C) | Dimethyl distearyl ammonium hectorite | 2.1 | 2.1 | 2.1 | 2.1 | 2.1 | 2.1 | 2.1 | 2.1 | 2.1 | 2.1 | 2.1 | 2.1 | 2.1 |
| Component (D) | PEG-10 Dimethicone*1 | 2.1 | - | - | - | 1.0 | - | - | - | 2.0 | - | 1.5 | - | - |
| | PEG-9 Polydimethylsiloxyethyl Dimethicone*2 | - | 2.5 | - | 1.0 | - | 2.0 | - | - | - | 1.0 | - | 2.5 | - |
| | Lauryl PEG-9 Polydimethylsiloxyethyl Dimethicone*3 | - | - | - | - | 1.0 | 2.0 | 3.0 | - | - | 1.0 | 1.5 | - | - |
| | Cetyl PEG/PPG-10/1 Dimethicone*4 | - | - | 3.0 | - | 1.0 | - | - | 3.0 | - | 1.0 | - | 2.5 | 4.0 |
| | (PEG-15/Laury 1 Dimethicone) Crosspolymer*5 | - | - | - | 1.5 | - | - | - | - | 1.0 | - | - | - | - |
| Component (A) | Dibutyl lauroyl glutamide | 0.3 | 0.6 | 0.6 | 1.2 | 1.2 | 3.6 | 3.6 | 4.1 | 4.1 | 5.4 | 16. 0 | 8.1 | 1.2 |
| Component (B) | Dibutyl ethylhexanoyl glutamide | 0.1 | 0.2 | 0.2 | 0.4 | 0.4 | 1.2 | 1.2 | 1.4 | 1.4 | 1.8 | 6.75 | 2.7 | 0.4 |
| Other oil agent | Isostearic acid | 0.6 | 1.2 | 1.2 | 2.4 | 2.4 | 7.2 | 7.2 | 9.5 | 9.5 | 12.8 | 2.25 | 19.2 | 2.4 |
| | Vaseline | 1.0 | 1.0 | 1.0 | 1.0 | 1.0 | 1.0 | 1.0 | 1.0 | 1.0 | 1.0 | 1.0 | 1.0 | 1.0 |

(continued)

| | Name of the raw materials | Working examples | | | | | | | | | | | | |
|---|---|---|---|---|---|---|---|---|---|---|---|---|---|---|
| | | 1 | 2 | 3 | 4 | 5 | 6 | 7 | 8 | 9 | 10 | 11 | 12 | 13 |
| Component (E) | Cyclopentasiloxane | 5.0 | - | - | 2.0 | - | 5.0 | - | - | 5.0 | - | - | 5.0 | 5.0 |
| | Dimethicone 5cs | - | 3.5 | 5.0 | 2.0 | 5.0 | 5.0 | - | 3.5 | - | 10. 0 | 10. 0 | 5.0 | - |
| | Dimethicone 10cs | - | - | 5.0 | - | - | - | 10. | - | - | - | - | 5.0 | - |
| | Dimethicone 20cs | - | - | - | - | 5.0 | - | - | - | - | - | - | - | - |
| | Dimethicone 50cs | - | - | - | 2.0 | - | - | - | - | - | - | - | 5.0 | - |
| | Dimethicone 100cs | - | - | - | - | - | - | - | 2.5 | - | 5.0 | - | - | - |
| | Diphenyl Dimethicone (TSF437) | - | - | - | 2.0 | - | - | - | - | - | - | - | - | - |
| | Dimethicone 1000cs | - | - | - | - | 5.0 | - | - | 5.0 | - | - | - | - | - |
| | Dimethicone 5000cs | - | - | - | - | - | 2.5 | - | - | - | - | - | - | - |
| | Dimethicone 10000cs | - | - | - | - | - | - | - | - | - | - | 2.5 | - | - |
| | Dimethicone 100000cs | - | - | - | - | - | 2.5 | - | - | 12. 0 | - | 2.5 | - | 5.0 |
| Component (F) | Cetyl 2-ethylhexanoate*6 | 12.0 | 6.0 | - | - | - | 5.0 | - | 2.5 | - | - | - | - | - |
| | Isononyl isononanoate*7 | - | 6.0 | 2.5 | 3.0 | 5.0 | 5.0 | - | 5.0 | 7.0 | - | 13.5 | 5.0 | 12.0 |
| | 2-ethylhexyl isononanoate* 8 | - | - | 2.5 | - | - | - | - | 2.5 | - | - | - | 5.0 | - |
| | Isodecyl isononanoate* 9 | - | - | - | - | 5.0 | - | 5.0 | - | - | 5.0 | - | - | - |
| | Isotridecyl isononanoate* 10 | - | - | - | 3.0 | - | - | 5.0 | - | - | - | - | - | - |
| | Isodecyl pivalate*11 | - | - | - | - | - | - | - | 2.5 | - | 5.0 | - | 5.0 | - |
| | 2-ethylhexyl succinate*12 | - | - | - | 2.0 | - | - | 5.0 | - | - | - | - | - | - |
| Stabilizer | Salt | 1.0 | 1.0 | 1.0 | 1.0 | 1.0 | 1.0 | 1.0 | 1.0 | 1.0 | 1.0 | 1.0 | 1.0 | 1.0 |

(continued)

| | Name of the raw materials | Working examples | | | | | | | | | | | | |
|---|---|---|---|---|---|---|---|---|---|---|---|---|---|---|
| | | 1 | 2 | 3 | 4 | 5 | 6 | 7 | 8 | 9 | 10 | 11 | 12 | 13 |
| Preservative | Phenoxyethanol | 0.3 | 0.3 | 0.3 | 0.3 | 0.3 | 0.3 | 0.3 | 0.3 | 0.3 | 0.3 | 0.3 | 0.3 | 0.3 |
| | Ratio of ester oil / silicone oil in external phase oil | 2.4 | 3.4 3 | 0.5 | 1.0 | 0.6 7 | 0.67 | 1.5 | 1.14 | 0.58 | 0.67 | 0.5 | 0.75 | 1.2 |
| | Hardness on the next day of the preparation | 0.013 | 0.2 | 0.29 | 0.39 | 0.4 2 | 0.74 | 0.5 | 0.59 | 0.85 | 0.44 | 0.98 | 0.93 | 0.83 |
| | Hardness after 1 month at 25°C from the preparation | 0.015 | 0.2 | 0.33 | 0.45 | 0.44 | 0.82 | 0.53 | 0.59 | 0.86 | 0.44 | 1.12 | 0.98 | 0.84 |
| | Appearance visual evaluation after 1 month at 50°C | ○ | ○ | ○ | ○ | ○ | ○ | ○ | ○ | ○ | ○ | ○ | ○ | ○ |
| From (In the form of cream or balm) | | Cream | Cream | Cream | Cream | Cream | Balm | Cream | Balm | Balm | Cream | Balm | Cream | Balm |
| Usability (Spreadabilit y on the skin) | | ◎ | ◎ | ◎ | ◎ | ◎ | ○ | ◎ | ○ | ○ | ◎ | ○ | ◎ | ◎ |
| Usability (Stickiness after the application) | | ○ | ◎ | ◎ | ◎ | ◎ | ◎ | ◎ | ◎ | ○ | ◎ | ○ | ◎ | ○ |
| Usability (Moistness 3 hours after the application) | | ◎ | ◎ | ○ | ◎ | ◎ | ◎ | ◎ | ◎ | ◎ | ◎ | ◎ | ◎ | ◎ |

[Table 2]

| | Name of the raw materials | Comparative examples | | | | | | | |
|---|---|---|---|---|---|---|---|---|---|
| | | 1 | 2 | 3 | 4 | 5 | 6 | 7 | 8 |
| Aqueous Component | Ion-exchanged water | Balance | Balance | Balance | Balance | Balance | Balance | Balance | Balance |
| | Glycerin | 13.0 | 13.0 | 13.0 | 13.0 | 13.0 | 13.0 | 13.0 | 13.0 |
| | 1,3-butylene glycol | 2.0 | 2.0 | 2.0 | 2.0 | 2.0 | 2.0 | 2.0 | 2.0 |
| | Polyethylene glycol#1540 | 1.0 | 1.0 | 1.0 | 1.0 | 1.0 | 1.0 | 1.0 | 1.0 |
| Component (C) | Dimethyl distearyl ammonium hectorite | 2.1 | - | 2.1 | 2.1 | 2.1 | 2.1 | 2.1 | - |
| Other oil thickening agent | Dextrin palmitate*13 | - | - | - | - | - | - | - | 2.1 |
| Component (D) | Polyether-modified silicone (PEG-10 Dimethicone)*1 | 2.1 | - | - | - | - | - | - | - |
| | Polyether-modified silicone (PEG-9 Polydimethylsiloxyethyl Dimethicone)*2 | - | 2.5 | - | - | 2.0 | - | - | - |
| | Polyether-modified silicone Lauryl PEG-9 Polydimethylsiloxyethyl Dimethicone)*3 | - | - | - | - | 2.0 | 3.0 | - | - |
| | Polyether-modified silicone (Cetyl PEG /PPG-10/1 Dimethicone)*4 | - | - | - | - | - | - | 3.0 | 2.1 |
| | Cross linked polyether-modified silicone (PEG-15/Lauryl Dimethicone) Crosspolymer*5 | - | - | - | - | - | - | - | - |
| Other emulsifier | Polyethyleneglycol Diisostearates(PEG-8)* 14 | - | - | - | 2.0 | - | - | - | - |
| Component (A) | Dibutyl lauroyl glutamide | - | 0.6 | 0.6 | 1.2 | 3.6 | 3.6 | 4.1 | 0.6 |
| Component (B) | Dibutyl ethylhexanoyl glutamide | - | 0.2 | 0.2 | 0.4 | 1.2 | 1.2 | 1.4 | 0.2 |
| Other oil agent | Isostearic acid | 0.6 | 1.2 | 1.2 | 2.4 | 7.2 | 7.2 | 9.5 | 1.2 |
| | Vaseline | 1.0 | 1.0 | 1.0 | 1.0 | 1.0 | 1.0 | 1.0 | 1.0 |

EP 3 659 579 A1

| | Name of the raw materials | Comparative examples | | | | | | | |
|---|---|---|---|---|---|---|---|---|---|
| | | 1 | 2 | 3 | 4 | 5 | 6 | 7 | 8 |
| Silicone oil | Cyclopentasiloxane | 5.0 | - | - | 2.0 | 10.0 | - | - | 5.0 |
| | Dimethicone 5cs | - | 3.5 | 5.0 | 2.0 | 10.0 | - | - | 5.0 |
| | Dimethicone 10cs | - | - | 5.0 | - | - | - | - | - |
| | Dimethicone 20cs | - | - | - | - | - | - | - | - |
| | Dimethicone 50cs | - | - | - | 2.0 | - | - | - | - |
| | Dimethicone 100cs | - | - | - | - | - | - | - | - |
| | Diphenyl Dimethicone(TSF437) | - | - | - | 2.0 | - | - | - | - |
| | Dimethicone 1000cs | - | - | - | - | - | - | - | - |
| | Dimethicone 5000cs | - | - | - | - | 2.5 | - | - | - |
| | Dimethicone 10000cs | - | - | - | - | - | - | - | - |
| | Dimethicone 100000cs | - | - | - | - | 2.5 | - | - | - |
| Ester oil | Cetyl 2-ethylhexanoate*6 | 12.0 | 6.0 | - | - | - | - | - | 6.0 |
| | Isononyl isononanoate*7 | - | 6.0 | 2.5 | 3.0 | - | 5.0 | - | 6.0 |
| | 2-ethylhexyl isononanoate*8 | - | - | 2.5 | - | - | - | - | - |
| | Isodecyl isononanoate*9 | - | - | - | - | - | 5.0 | - | - |
| | Isotridecyl isononanoate*10 | - | - | - | 3.0 | - | 5.0 | - | - |
| | Isodecyl pivalate*11 | - | - | - | - | - | - | - | - |
| | 2-ethylhexyl succinate*12 | - | - | - | 2.0 | - | 5.0 | - | - |
| Other oil agent | Isododecane | - | - | - | - | - | - | 10.0 | - |
| | Isohexadecane | - | - | - | - | - | - | 5.0 | - |
| Stabiliz er | Salt | 1.0 | 1.0 | 1.0 | 1.0 | 1.0 | 1.0 | 1.0 | 1.0 |
| Preserva tive | Phenoxyethanol | 0.3 | 0.3 | 0.3 | 0.3 | 0.3 | 0.3 | 0.3 | 0.3 |

EP 3 659 579 A1

(continued)

| | Name of the raw materials | Comparative examples | | | | | | | |
|---|---|---|---|---|---|---|---|---|---|
| | | 1 | 2 | 3 | 4 | 5 | 6 | 7 | 8 |
| | Ratio of ester oil / silicone oil in external phase oil | 2.4 | 3.43 | 0.5 | 1.0 | 0.67 | 1.5 | - | 1.2 |
| | Hardness on the next day of the preparation | 0.01 3 | 0.2 | Could not be prepared | 0.39 | 0.74 | 0.5 | 0.21 | 0.35 |
| | Hardness after 1 month at 25°C from the preparation | - | 0.2 | - | 0.45 | 0.82 | 0.53 | 0.23 | 0.35 |
| | Appearance visual evaluation after 1 month at 50 °C | Separated | Separated | - | Separated | ○ | ○ | ○ | ○ |
| | From (In the form of cream or balm) | Cream | Cream | - | Cream | Balm | Cream | Cream | Cream |
| Usability (Spreadability on the skin) | | - | - | - | - | ○ | × | ◎ | Δ |
| Usability (Stickiness after the application) | | - | - | - | - | ◎ | × | ◎ | Δ |
| Usability (Moistness 3 hours after the application) | | - | - | - | - | Δ | ◎ | × | ○ |

The following ingredients were used for *1 to *13 in the above Table 1 and Table 2.

*1: KF-6017P; Shin-Etsu Chemical Co., Ltd.
*2: KF-6028P; Shin-Etsu Chemical Co., Ltd.
*3: KF-6038; Shin-Etsu Chemical Co., Ltd.
*4: KF-6048; Shin-Etsu Chemical Co., Ltd.
*5: KSG-320; Shin-Etsu Chemical Co., Ltd.
*6: CEH; Kokyu Alcohol Kogyo Co. Ltd.
*7: KAK99; Kokyu Alcohol Kogyo Co. Ltd.
*8: ES108109; Kokyu Alcohol Kogyo Co. Ltd.
*9: KAK109; Kokyu Alcohol Kogyo Co. Ltd.
*10: KAK139; Kokyu Alcohol Kogyo Co. Ltd.
*11: NEOLIGHT 100P; Kokyu Alcohol Kogyo Co. Ltd.
*12: KAK DIOS; Kokyu Alcohol Kogyo Co. Ltd.
*13: Rheopearl KL; Chiba Flour Milling Co., Ltd.
*14: EMALEX 400di-ISEX; Nihon Emulsion Co., Ltd.

From the above results, the following points can be understood.

When it does not comprise the components (A) and (B), the emulsion is separated and thus a stable emulsion cannot be provided.

When it does not comprise the component (C), the emulsion is also separated and thus a stable emulsion cannot be provided. Further, even if other oil thickening agent is employed instead of this component to stabilize the emulsion, such emulsion is not preferable in term of spreadability on the skin and stickiness after the application, and thus an emulsion with excellent feeling of use cannot be provided.

When it does not comprise the component (D), an emulsion cannot be prepared, and even if other emulsifier is used instead of it, the emulsion is separated and thus a stable emulsion cannot be provided.

When it does not comprise the component (E), a stale emulsion can be obtained, however, this emulsion is not preferable in term of spreadability on the skin and stickiness after the application, and thus an emulsion with excellent feeling of use cannot be provided.

When it does not comprise the component (F), a stale emulsion can be obtained, however, this emulsion is not preferable in term of moistness after the application, and thus an emulsion with excellent feeling of use cannot be provided.

When an oil agent other than the components (E) and (F) is employed, a stale emulsion can be obtained, however, an emulsion with excellent feeling of use cannot be provided.

[Example 14]

An anti-aging cream of the formulation shown in Table 3 was prepared by the following method.

[Table 3]

| [Example 14] Anti-aging cream | | | Blending amount (mass%) |
|---|---|---|---|
| (1) | Ion-exchanged water | | Balance |
| (2) | Glycerin | | 5.0 |
| (3) | Dipropylene glycol | | 7.0 |
| (4) | 1,3-butylene glycol | | 3.0 |
| (5) | Polyethylene glycol 6000 | | 2.0 |
| (6) | Component (C) Dimethyl distearyl ammonium hectorite / Propylene carbonate / Isododecane mixture Product name:BENTONE GEL ISD V,ELEMENTIS | | 2.5 |
| (7) | Component (D) PEG-10 Dimethicone Product name:ES-5612,Dow Corning Toray Co.,Ltd. | | 2.5 |
| (8) | Component (A) Dibutyl lauroyl glutamide | | 0.6 |
| (9) | Component (B) Dibutyl ethylhexanoyl glutamide | | 0.2 |
| (10) | Isostearic acid | | 1.2 |
| (11) | Isododecane | | 0.5 |
| (12) | Component (E) Dimethicone 6cs | | 5 |

(continued)

| [Example 14] Anti-aging cream | | | Blending amount (mass%) |
|---|---|---|---|
| (13) | Component (F) Isononyl isononanoate | | 10.0 |
| | Product name:KAK 99, Kokyu Alcohol Kogyo Co. Ltd. | | |
| (14) | Component (F) Isostearyl pivalate | | 3.5 |
| | Product name: NEOLIGHT 180P; Kokyu Alcohol Kogyo Co. Ltd. | | |
| (15) | Carnosine | | 1.0 |
| (16) | Piperidine propionic acid | | 1.0 |
| (17) | Vitamin E-acetate | | 0.5 |
| (18) | Trisodium edetate | | 0.1 |
| (19) | Salt | | 1.0 |
| (20) | Fragrance | | q.s. |

```
Ratio of Ester oil / Silicone oil = 2.7
```

<Production method>

(6) to (14) and (17) were uniformly dissolved at 110 °C (an oil phase). Subsequently, an aqueous phase was prepared by uniformly dissolving (1) to (5) and (15) (16) (18) (19) at 80 °C.

While stirring the oil phase at 80 °C with a disperser, the aqueous phase was gradually added and emulsified. When the emulsification was completed, (20) was added, and the mixture was stirred again with a disperser to obtain an anti-aging cream having a desired hardness of 0.35. The obtained anti-aging cream had stability over time and was also excellent in the feeling of use.

[Example 15]

A whitening balm of the formulation shown in Table 4 was prepared by the following method.

[Table 4]

| [Working example 15] Whitening Balm | | Blending amount (mass%) |
|---|---|---|
| (1) | Ion-exchanged water | Balance |
| (2) | Glycerin | 5.0 |
| (3) | Dipropylene glycol | 7.0 |
| (4) | 1,3-butylene glycol | 3.0 |
| (5) | Polyethylene glycol 20000 | 2.0 |
| (6) | Component (C) Dimethyl distearyl ammonium hectorite | 2.5 |
| | Product name:BENTONE 38VCG, ELEMENTIS | |
| (7) | Component (D) Polyether-modified silicone | 2.5 |
| | Product name:KF-6048,Shin-Etsu Chemical Co., Ltd. | |
| (8) | Component (A) Dibutyl lauroyl glutamide | 3.6 |
| (9) | Component (B) Dibutyl ethylhexanoyl glutamide | 1.2 |
| (10) | Isostearic acid | 7.2 |
| (11) | Vaseline | 1.0 |
| (12) | Dimethicone 5cs | 5.0 |
| (13) | Isononyl isononanoate | 6.0 |
| | Product name: KAK 99, Kokyu Alcohol Kogyo Co. Ltd. | |
| (14) | 2-ethylhexyl isononanoate | 5.0 |
| | Product name: NEOLIGHT 180P; Kokyu Alcohol Kogyo Co. Ltd. | |
| (15) | Potassium 4-methoxysalicylate | 1.0 |
| (16) | Tranexamic acid | 1.0 |
| (17) | Green tea extract | 0.1 |

(continued)

| [Working example 15] Whitening Balm | | Blending amount (mass%) |
|---|---|---|
| (18) | Trisodium edetate | 0.1 |
| (19) | Salt | 1.0 |
| (20) | Fragrance | q.s. |

```
Ratio of Ester oil / Silicone oil  = 2.2
```

<Production method>

(6) to (14) and (18) (20) were uniformly dissolved at 110 °C (an oil phase). Subsequently, an aqueous phase is prepared by uniformly dissolving (1) to (5) and (15) (16) (17) (19) at 80 °C.

While stirring the oil phase at 80 °C with a disperser, the aqueous phase was gradually added and emulsified. When the emulsification was completed, (20) was added and the mixture was stirred again with a disperser to obtain the whitening balm having a desired hardness of 0.95. The obtained whitening balm had stability over time and was excellent in the feeling of use.

**[Industrial Applicability]**

As described above, it is possible to provide a W/O type emulsion having excellent usability and storage stability.

Such emulsion can be used for applications such as cosmetics.

**Claims**

1.  A W/O type emulsion comprising

    Component (A): dibutyl lauroyl glutamide,
    Component (B): dibutyl ethylhexanoyl glutamide,
    Component (C): an organically modified clay mineral,
    Component (D): a polyether-modified silicone,
    Component (E): a silicone oil, and
    Component (F): an ester oil.

2.  The emulsion according to Claim 1 comprising 5% by mass or more of the ester oil.

3.  The emulsion according to Claim 1 or 2, wherein the mass ratio of the ester oil / the silicone oil is 0.5 or more.

4.  The emulsion according to Claim 3, wherein the mass ratio of the ester oil / the silicone oil is 1.0 or more.

5.  The emulsion according to any one of Claims 1 to 4 comprising 3.5% by mass or more of the silicone oil.

6.  The emulsion according to any one of Claims 1 to 5, wherein the component (D) comprises a non-crosslinked polyether-modified silicone.

7.  The emulsion according to any one of Claims 1 to 6 comprising

    0.1 to 20.0% by mass of the component (A),
    0.1 to 10.0% by mass of the component (B),
    0.5 to 5.0% by mass of the component (C), and
    0.5 to 5.0% by mass of the component (D).

8.  The emulsion according to any one of Claims 1 to 7 which is in the form of balm.

9.  The emulsion according to any one of Claims 1 to 8 which is a cosmetic.

**INTERNATIONAL SEARCH REPORT**

| International application No. |
| --- |
| PCT/JP2018/027606 |

**A. CLASSIFICATION OF SUBJECT MATTER**
Int.Cl. A61K8/44(2006.01)i, A61K8/06(2006.01)i, A61K8/19(2006.01)i,
 A61K8/25(2006.01)i, A61K8/37(2006.01)i, A61K8/891(2006.01)i,
 A61K8/894(2006.01)i, A61Q19/00(2006.01)i
According to International Patent Classification (IPC) or to both national classification and IPC

**B. FIELDS SEARCHED**

Minimum documentation searched (classification system followed by classification symbols)
Int.Cl. A61K8/44, A61K8/06, A61K8/19, A61K8/25, A61K8/37, A61K8/891, A61K8/894,
 A61Q19/00

Documentation searched other than minimum documentation to the extent that such documents are included in the fields searched
Published examined utility model applications of Japan 1922–1996
Published unexamined utility model applications of Japan 1971–2018
Registered utility model specifications of Japan 1996–2018
Published registered utility model applications of Japan 1994–2018

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2004/024798 A1 (SHIN-ETSU CHEMICAL CO., LTD.) 25 March 2004, page 47, line 20 to page 48, line 22, example 23 & US 2006/0034875 A1, paragraphs [0222]–[0228], example 23 & EP 1550687 A1 & KR 10-2005-0042199 A | 1–9 |
| A | WO 2014/054686 A1 (DOW CORNING TORAY CO., LTD.) 10 April 2014, paragraphs [0122]–[0124], treatment example 1 & US 2015/0239924 A1, paragraphs [0144]–[0146], treatment example 1 & JP 2014-70220 A & EP 2905300 A1 & KR 10-2015-0064156 A & CN 104769015 A | 1–9 |

☒ Further documents are listed in the continuation of Box C. ☐ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 19 October 2018 (19.10.2018) | 30 October 2018 (30.10.2018) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
|---|
| PCT/JP2018/027606 |

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2012-149052 A (DOW CORNING TORAY CO., LTD.) 09 August 2012, paragraphs [0231]-[0232], treatment example 10 & US 2014/0004065 A1, paragraphs [0242]-[0243], treatment example 10 & WO 2012/091155 A1 & CN 103298857 A & KR 10-2013-0102635 A | 1-9 |
| A | JP 2011-116701 A (DOW CORNING TORAY CO., LTD.) 16 June 2011, paragraph [0129], treatment example 5 & US 2012/0237583 A1, paragraphs [0158]-[0160], treatment example 5 & WO 2011/068250 A1 & CN 102639542 A & KR 10-2012-0096543 A | 1-9 |
| A | JP 2014-507264 A (AVON PRODUCTS, INC.) 27 March 2014, paragraphs [0004]-[0007] & US 2012/0164093 A1, paragraphs [0004]-[0007] & WO 2012/091823 A1 & CN 103298458 A | 1-9 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H08268831 A
- JP 2002275028 A
- WO 2012091823 A